# FASCICULE DE BREVET EUROPEEN

(11) **EP 0 780 379 B1**
(45) Date de publication et mention de la délivrance du brevet: **22.04.1998**
(21) Numéro de dépôt: 96402661.1
(22) Date de dépôt: 09.12.1996
(51) Int. Cl.: C07D 233/34, A61K 31/415

(54) **Nouveau dérivé de 4-imidazolidinone et son utilisation comme agoniste du récepteur de NMDA**
Ein 4-Imidazolinon-Derivat und deren Verwendung als NMDA-Rezeptor-Agonist
A 4-imidazolidinone derivative and its use as NMDA receptor agonist

(30) Priorité: 21.12.1995 FR 9515223
(43) Date de publication de la demande: 25.06.1997
(73) Titulaire: ADIR ET COMPAGNIE, 92415 Courbevoie Cédex (FR)
(72) Inventeur: Cordi, Alex, 92150 Suresnes (FR); Lacoste, Jean-Michel, 92310 Sevres (FR); Millan, Mark, 78230 Le Pecq (FR); Audinot, Valérie, 78300 Poissy (FR)

(56) Documents cités:
- EP-A- 0 319 824

## Description

La présente invention concerne un nouveau dérivé de 4-imidazolidinone, son procédé de préparation et les compositions pharmaceutiques qui le contiennent.

Le composé de la présente invention, outre le fait qu'il soit nouveau, est un puissant agoniste partiel du site réceptoriel glycine couplé au récepteur N-méthyl-D-aspartate (NMDA) (récepteur glycine B).

L'acide L-glutamique et l'acide L-aspartique ont la capacité d'activer les neurones du système nerveux central et de nombreux travaux ont démontré que ces aminoacides excitateurs (AAE) répondent aux critères définissant un neurotransmetteur. C'est pourquoi la modulation des événements neuronaux liés à ces AAE apparait être une cible intéressante pour le traitement des maladies neurologiques et psychiatriques.

Parmi les 4 groupes de récepteurs aux AAE à localisation post- et pré-synaptique, le récepteur NMDA est associé à un canal ionique perméable aux cations mono- et divalents (dont le calcium) mais qui est bloqué par le magnésium. L'ouverture du canal NMDA est régulée par plusieurs sites associés au récepteur et en particulier est favorisée par la glycine dont l'effet est strychnine-insensible. Ce site glycine constitue l'une des cibles importantes pour moduler l'activation du récepteur NMDA.

La modulation positive de la transmission NMDA en agissant en tant qu'agoniste au niveau des récepteurs glycine B est un moyen d'améliorer le fonctionnement de l'apprentissage et la mémoire en général (J.B. Monahan et coll., Pharmacol. Biochem. & Behavior, 34, 649-653, 1989 ; W.E. MÜLLER et coll., Life Sciences, 55, n° 25/26, 2147/2153, 1994), ainsi que ses dysfonctionnements liés au processus de vieillissement, des maladies neurodégénératives, les démences comme la maladie d'Alzheimer, la maladie de Pick, la chorée de Huntington, la schizophrénie ou les états anxio-dépressifs (A. HASHIMOTO et coll., J. Neurochem, 60, n° 2, 783-786, 1993 ; P. SARANSAARI, Mechanisms of Ageing and Devel., 72, 57-66, 1993). De plus, une activité agoniste ou agoniste partielle du type glycine B pourrait être utile dans le traitement des convulsions, par exemple celles associées à l'épilepsie (M.G. BAXTER et Coll., CNS Drug Reviews, 1, n° 1, 74-90, 1995 ; D.O. NORRIS et coll., Pharmacol. Biochem. & Behavior, 43, 609-612, 1992), dans le traitement de la douleur (A.H. DICKENSON et Coll., Neuroscience Lett., 121, 263-266, 1991 ; M.J. MILLAN et Coll., Europ. J. Pharmacol., 238, 445-447, 1993), dans le contrôle des symptômes productifs et déficitaires de la schizophrénie (M. ISHIMARU et coll., Biol. Psychiatry, 35, 84-95, 1994 ; A.O. SHERMAN et coll., Biol. Psychiatry, 30, 1191-1198, 1991), dans le traitement de l'anxiété (R. TRULLAS et coll., Europ. J. Pharmacol., 203, 379-385, 1991 ; J.T. WINSLOW et coll., Europ. J. Pharmacol., 190, 11-21, 1990) et de la dépression (I.A. PAUL et coll., Psychopharm., 106, 285-287, 1992 ; R. TRULLAS cité plus haut) et dans le traitement des problèmes associés avec l'abus de drogues tels que l'alcool (C.R. BREESE et coll., Brain Research, 674, 82-90, 1995 ; S.J. DEUTSCH et coll., Clinical. Neuropharmacol., 12, n° 6, 483-489, 1989) et les psychostimulants (G.E. EVONIUK et coll., Psychopharm., 105, 125-128, 1991 ; E. TOTH et Coll., Neurochem. Research, 11, n° 3, 393-400, 1986).

Des dérivés simples de 4-imidazolidinones ont rarement été décrits dans la littérature. On peut néanmoins citer les composés décrits par R.E. HARMON et coll. (J. Het. Chem., 70, vol. 7 (2), p. 439-442).

Aucune activité pharmacolologique particulière n'a été décrite pour ces composés dans l'Art Antérieur.

Plus spécifiquement, la présente invention concerne le 3-hydroxy-4-imidazolidinone de formule (I) : 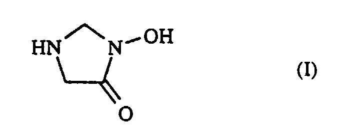 ainsi que ses sels d'addition à un acide ou à une base pharmaceutiquement acceptable.

Parmi les acides pharmaceutiquement acceptables, on peut citer à titre d'exemple non limitatif les acides chlorhydrique, bromhydrique, sulfurique, phosphorique, acétique, trifluoroacétique, lactique, pyruvique, malonique, succinique, glutarique, fumarique, tartrique, maléïque, citrique, ascorbique, oxalique, méthane sulfonique, camphorique, etc...

Parmi les bases pharmaceutiquement acceptables, on peut citer à titre non limitatif l'hydroxyde de sodium, l'hydroxyde de potassium, la triéthylamine, la tertbutylamine, etc...

La présente invention concerne également le procédé d'obtention de ce composé caractérisé en ce que l'on fait réagir l'acide glycine hydroxamique de formule (II) :

H₂N-CH₂-CO-NH-OH (II)

avec une solution aqueuse de formaldéhyde,
pour conduire au composé de formule (I), que l'on purifie, le cas échéant, selon une technique classique de purification et que l'on transforme, si on le souhaite, en ses sels d'addition à un acide ou à une base pharmaceutiquement acceptable.

La présente invention a également pour objet les compositions pharmaceutiques renfermant comme principe actif le composé de formule (I) seul ou en combinaison avec un ou plusieurs excipients ou véhicules inertes non toxiques.

Parmi les compositions pharmaceutiques selon l'invention, on pourra citer plus particulièrement celles qui conviennent pour l'administration orale, parentérale, nasale, les comprimés simples ou dragéifiés, les comprimés sublinguaux, les gélules, les tablettes, les suppositoires, les crèmes, pommades, gels dermiques, etc...

La posologie utile varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 1 et 1000 mg pour un traitement en 1 à 3 prises par 24 heures.

Les exemples suivants illustrent l'invention et ne la limitent en aucune façon.

La structure du composé de l'invention a été confirmée par les techniques spectroscopiques usuelles (résonance magnétique nucléaire, infrarouge, spectrométrie de masse, diffraction de rayons X,...).

### EXEMPLE 1 : 3-Hydroxy-4-imidazolidinone

A une solution maintenue sous atmosphère d'azote contenant 11 mmoles d'acide glycine hydroxamique dans 50 ml d'éthanol, est additionnée, en une fois, à 20°C, 0,9 ml d'une solution aqueuse à 40 % de formaldéhyde. Le mélange est ensuite chauffé à reflux pendant 3 heures puis refroidi à 20°C et agité à cette température pendant une nuit. Le résidu cristallin obtenu est essoré, lavé par de l'éthanol puis de l'éther éthylique. Le produit attendu est alors purifié par cristallisation dans de l'éthanol.
*Point de fusion : 147-148°C*

| *Microanalyse élémentaire :* | | | |
|---|---|---|---|
| | *C %* | *H %* | *N %* |
| *calculé* | *35,29* | *5,92* | *27,44* |
| *trouvé* | *35,21* | *5,72* | *27,08* |

### ETUDE PHARMACOLOGIQUE DU COMPOSE DE L'INVENTION

### EXEMPLE 2 : Expériences de liaison de la [³H]-glycine et du [³H]-MK 801 sur des membranes de cerveau de rat

### Matériel et méthodes

### Préparation des membranes (d'après Yoneda et coll., J. Neurochem, 55, 1, 237-244, 1990)

Les membranes sont préparées et utilisées de la même façon pour les deux radioligands. Les tampons sont préparés avec de l'eau Milli-Q (Millipore) stérile, déionisée et filtrée, juste avant utilisation sur filtre de nitrocellulose (0,45 µm).
Après dissection, les cerveaux de rats (mâles Wistar, 240-260 g), débarasssés du cervelet, sont placés dans un tampon glacé Tris-acétate (1 mM), EGTA (1 mM), sucrose (320 mM) à pH 7. Les cerveaux sont broyés puis homogénéisés à l'aide d'un polytron. La suspension est centrifugée 10 minutes à 1000 g, le surnageant contenant les membranes est récupéré et mis à nouveau à centrifuger 20 minutes, à 35 000 g. Le culot, débarassé d'un anneau brun, est récupéré dans un tampon de lyse Tris-acétate (1 mM), EGTA (1 mM) à pH 8. La suspension est alors laissée 15 minutes à 4°C, puis est centrifugée 20 minutes à 35 000 g. Le culot est récupéré dans un tampon Tris-acétate (50 mM) à pH 7,4, contenant 0,08 % de Triton et laissé 30 minutes à 4°C. Après deux centrifugations successives, le culot est repris dans du tampon Tris-acétate (50 mM) à pH 7,4, aliqoté et stocké à -80°C. Le jour de l'expérience, au maximum 3 semaines après la préparation, les membranes sont lavées deux fois avec le tampon Tris-acétate (50 mM) à pH 7,4.

*Expériences de liaison (d'après Yoneda et coll., J. Neurochem., 60,* 2, *634-645, 1993)*

| **Site** | **Radioligand** | **Liaison non spécifique** | **Incubation** |
|---|---|---|---|
| Glycine B | [³H]-Glycine (10nM) | Glycine (10 mM) | 20 minutes à 4°C |
| Canal NMDA | [³H]-MK 801 (1 nM) | PCP (10 µM) | 120 minutes à 20°C |

La séparation des radioligands libre et lié est effectuée sur filtres GF/B préalablement imbibés de PEI (0,1 %) à l'aide d'un appareil de filtration Brandle. Dans les deux cas, la filtration est effectuée très rapidement en utilisant du tampon glacé Tris-acétate (50 mM) à pH 7,4 (contenant 10 mM de MgSO₄ pour les expériences de [³H]-Glycine). Chaque filtre est rincé 3 fois. La radioactivité est mesurée grâce à un compteur β (Tricarb 1500, Packard) et exprimée en dpm. Les courbes obtenues sont analysées à l'aide du logiciel GraphPad Prism (régression non linéaire).

### Résultats (Tableau 1)

La liaison au site glycine B reflète l'affinité (IC₅₀) des produits glycinergiques pour ce site. La glycine, ligand agoniste endogène, l'agoniste D-Serine, également supposé être un agoniste endogène (A. HASHIMOTO et coll., J. Neurochem., 60, n° 2, 783-786, 1993) et l'antagoniste L-701,324 présentent respectivement des afffinités de l'ordre de 0,247 µM, 0,673 µM et 0,026 µM .

Le composé de l'invention présente une affinité similaire à celle de l'agoniste partiel D-Cycloserine et de l'antagoniste (+) HA 966, soit respectivement 6,8, 7,4 et 7,3 µM.

L'ouverture du canal du complexe réceptoriel NMDA est modulée positivement par le site glycinergique. Le MK 801 est un ligand agissant en se fixant à l'intérieur du canal. La liaison du [³H]-MK801 est donc stimulée par un agoniste et inhibée par un antagoniste glycinergique. C'est ainsi que, comme la glycine, la D-Serine et la D-Cycloserine, le composé de l'invention stimule la liaison du [³H]-MK 801. Il est à noter que pour les agonistes, les EC₅₀ obtenues en liaison [³H]-MK 801 sont en général inférieures aux IC₅₀ obtenues en liaison [³H-]glycine. Au niveau de l'efficacité, la glycine exerce un effet stimulateur maximum de 59 %, effet qui se rapproche à celui de la D-Serine, tandis que la D-Cycloserine, un agoniste partiel, n'exerce qu'un effet égal à 22 %. Le composé de l'invention se comporte comme un agoniste dont l'efficacité (46 %) est nettement supérieure à celle de la D-Cycloserine, mais légèrement inférieure à celle de la glycine et de la D-Serine. En ce qui concerne les antagonistes, l'effet inhibiteur obtenu est quasi maximal.

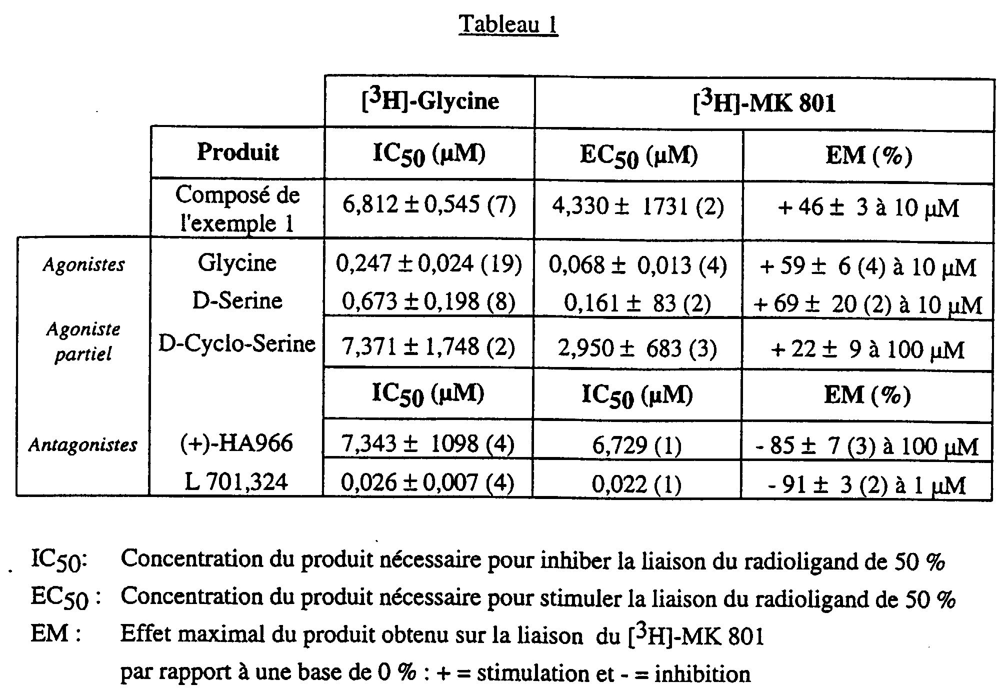

### EXEMPLE 3 : Composition pharmaceutique

| Formule de préparation pour 1000 comprimés dosés à 10 mg | |
|---|---|
| Composé de l'exemple 1 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composé de formule (I) : 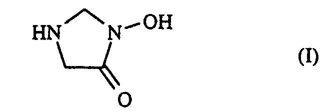 ainsi que ses sels d'addition à un acide ou à une base pharmaceutiquement acceptable.

2. Procédé de préparation du composé de formule (I) selon la revendication 1, caractérisé en ce que l'on fait réagir l'acide glycine hydroxamique de formule (II) :
H₂N-CH₂-CO-NH-OH (II)
avec une solution aqueuse de formaldéhyde,
pour conduire au composé de formule (I), que l'on purifie, le cas échéant, selon une technique classique de purification et que l'on transforme, si on le souhaite, en ses sels d'addition à un acide ou à une base pharmaceutiquement acceptable.

3. Compositions pharmaceutiques contenant comme principe actif le composé de formule (I) selon la revendication 1, seul ou en combinaison avec un ou plusieurs excipients ou véhicules inertes, non toxiques, pharmaceutiquement acceptables.

4. Compositions pharmaceutiques selon la revendication 3 contenant le principe actif selon la revendication 1 utile comme agoniste partiel du site glycine B.

## Patentansprüche

1. Verbindung der Formel (I): 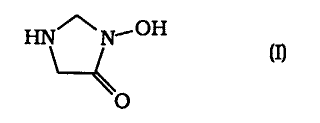 sowie deren Additionssalze mit einer pharmazeutisch annehmbaren Säure oder Base.

2. Verfahren zur Herstellung der Verbindung (I) nach Anspruch 1, dadurch gekennzeichnet, daß man Glycinhydroxamsäure der Formel (II):
H₂N - CH₂ - CO - NH - OH (II)
mit einer wäßrigen Formaldehydlösung umsetzt
zur Bildung der Verbindung der Formel (I), welche man gegebenenfalls mit Hilfe einer klassischen Reinigungsmethode reinigt oder gewünschtenfalls in ihre Additionssalze mit einer pharmazeutisch annehmbaren Säure oder Base umwandelt.

3. Pharmazeutische Zubereitungen enthaltend als Wirkstoff die Verbindung der Formel (I) nach Anspruch 1 allein oder in Kombination mit einem oder mehreren inerten, nichttoxischen, pharmazeutisch annehmbaren Trägermaterialien oder Bindemitteln.

4. Pharmazeutische Zubereitungen nach Anspruch 3 enthaltend den Wirkstoff nach Anspruch 1 als Teilagonist für die Bindungsstelle für Glycin B.

## Claims

1. Compound of formula (I): 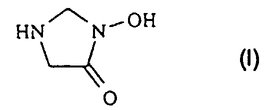 and its addition salts with a pharmaceutically acceptable acid or base.

2. Process for the preparation of a compound of formula (I) according to claim 1, characterised in that glycine hydroxamic acid of formula (II):
H₂N-CH₂-CO-NH-OH (II)
is reacted with an aqueous formaldehyde solution,
to yield a compound of formula (I) which, where appropriate, is purified according to a conventional purification method and, if desired, converted into addition salts thereof with a pharmaceutically acceptable acid or base.

3. Pharmaceutical compositions comprising as active ingredient a compound of formula (I) according to claim 1, on its own or in combination with one or more pharmaceutically acceptable, inert, non-toxic excipients or carriers.

4. Pharmaceutical compositions according to claim 3 comprising an active ingredient according to claim 1 for use as a partial agonist of the glycine B site.
